(19) Europäisches Patentamt / European Patent Office / Office européen des brevets

(11) **EP 4 650 782 A1**

(12) **EUROPEAN PATENT APPLICATION**
published in accordance with Art. 153(4) EPC

(43) Date of publication:
**19.11.2025 Bulletin 2025/47**

(21) Application number: **24741588.8**

(22) Date of filing: **12.01.2024**

(51) International Patent Classification (IPC):
***G01N 33/86*** *(2006.01)*

(52) Cooperative Patent Classification (CPC):
**G01N 33/86**

(86) International application number:
**PCT/JP2024/000586**

(87) International publication number:
**WO 2024/150816 (18.07.2024 Gazette 2024/29)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **13.01.2023 JP 2023003980**

(71) Applicant: Sekisui Medical Co., Ltd.
**Tokyo 103-0027 (JP)**

(72) Inventors:
• KAWABE, Toshiki
**Tokyo 103-0027 (JP)**
• ONISHI, Kengo
**Tokyo 103-0027 (JP)**

(74) Representative: **Wächtershäuser & Hartz
Patentanwaltspartnerschaft mbB
Weinstraße 8
80333 München (DE)**

(54) **METHOD FOR ANALYZING BLOOD COAGULATION REACTION**

(57) A method for analyzing a blood coagulation reaction includes: (1) acquiring P(i) which is a blood coagulation reaction of a blood specimen, where i is a variable indicating the number of measurement points or time; (2) acquiring t1 and t2 from the P(i), where both t1 and t2 represent the number of measurement points or time, t1 = i, and t2 < t1, and t1 and t2 satisfy an equation: P(t2) = P(t1) × X%, where the P(t1) and the P(t2) respectively represent blood coagulation reactions of the blood specimen at t1 and t2, and X ranges from 20 to 70; and (3) acquiring Rt which represents a ratio of t1 to t2.

**EP 4 650 782 A1**

## Description

Technical Field

The present invention relates to a method for analyzing a blood coagulation reaction.

Background Art

A blood coagulation test is for diagnosing the blood coagulation ability of a subject by checking the coagulation reaction of a blood specimen of the subject. Typically, in the blood coagulation test, a predetermined reagent is added to the blood specimen, subsequently, its coagulation reaction is measured over time, and a blood coagulation time is calculated based on measured data of the acquired coagulation reaction. Conventionally, the blood coagulation time is used as an indicator for detecting an error in the blood coagulation ability.

The measured data of the coagulation reaction includes various noises due to the states of a reaction measurement part, a reagent, and a specimen. The noise may cause erroneous detection of the coagulation time. For example, a phenomenon called an early reaction error, which is an increase of the coagulation reaction curve before a time point at which a true coagulation reaction is supposed to begin sometimes occurs. It is required to reduce the adverse effects due to noise in the measured data of the coagulation reaction, and correctly evaluate the coagulation reaction.

Patent Literature 1 describes a method for analyzing a blood coagulation reaction characterized by providing a checkpoint or a check region in a period from the initiation of measuring the blood coagulation reaction to the end of the coagulation reaction, and monitoring the reaction state at the checkpoint or in the check region, thereby detecting an early reaction error. Patent Literature 2 describes setting a noise determination line, based on the relationship between the coagulation time and the scattered light intensity measured at the time, determining the coagulation time for the test specimen to be normal if the scattered light intensity at the test specimen at the coagulation time exceeds the noise determination line, and continuing the measurement of the coagulation reaction until a normal coagulation time is acquired if not, thereby preventing the coagulation time from being erroneously detected due to noise. Patent Literature 3 describes determination of an error in a coagulation reaction, based on the ratio of the difference (T[i + 1] - T[i]) between time (T[i]) corresponding to the coagulation rate (i) and time (T[i + 1]) corresponding to the coagulation rate (i + 1) to time (from SP to EP) from the coagulation initiation point to the coagulation end point.

In recent years, automatic analysis devices for coagulation reaction measurement have generally been used. In a blood coagulation test by a conventional automatic analysis device, typically, the coagulation reaction is measured until the end of the coagulation reaction, and the coagulation time is calculated based on acquired measured data. Accordingly, in a case of measuring the coagulation reactions of a large number of blood specimens by the automatic analysis device, to prevent measurement from being terminated midway through the coagulation reaction, it is typical to set a longer measurement time so as to allow measured data to be collected until the end of the coagulation reaction of an abnormal specimen with a prolonged coagulation time. On the other hand, most of the specimens to be measured are normal specimens. Accordingly, the longer the measurement time is set, the lower the entire analytical efficiency becomes.

Methods for optimizing the time required to analyze the coagulation reaction of each specimen by detecting the end of the coagulation reaction in real time while measuring the coagulation reaction, and by calculating the coagulation time have been developed. Patent Literature 4 describes a method for measuring the coagulation reaction based on the accumulated ratio Z of the coagulation reaction in a minute time period while detecting the end of the coagulation reaction in real time, and calculating the coagulation time. Patent Literature 5 describes a method for measuring the coagulation reaction while acquiring the reaction rate V of the coagulation reaction in real time, and calculating the coagulation time at a time point when the acquired V becomes S% of the maximum of the V acquired so far.

Citation List

Patent Literature

Patent Literature 1: JP-A-2003-169700
Patent Literature 2: JP-A-2010-217059
Patent Literature 3: JP-A-2007-107889
Patent Literature 4: WO 2021/132552
Patent Literature 5: WO 2021/206107

Summary of Invention

Technical Problem

The present invention provides a method that can distinguish an early reaction error included in the measured data of the blood coagulation reaction and a true coagulation reaction from each other, and allows more correct analysis of the coagulation reaction.

Solution to Problem

That is, the present invention provides the following.

[1] A method for analyzing a blood coagulation reaction, the method comprising:

(1) acquiring P(i) which is a blood coagulation reaction of a blood specimen, where i denotes the number of measurement points or time;
(2) acquiring t1 and t2 from the P(i),

where both t1 and t2 represent the number of measurement points or time, t1 = i, and t2 < t1, and t1 and t2 satisfy an equation: P(t2) = P(t1) × X%, where

the P(t1) and the P(t2) respectively represent blood coagulation reactions of the blood specimen at t1 and t2, and
X ranges from 20 to 70; and

(3) acquiring Rt which represents a ratio of t1 to t2.

[2] The method according to [1], further comprising evaluating whether the P(i) is a true blood coagulation reaction or not based on the Rt.
[3] The method according to [1], further comprising evaluating that the P(i) is an early reaction when the Rt is greater than a predetermined value.
[4] The method according to any one of [1] to [3], further comprising repeating steps from the (1) to (3) with i = i + k (where k > 0) until the Rt reaches or becomes smaller than a predetermined value to newly acquire P(i), t1, t2, and Rt.
[5] The method according to [4], further comprising repeating acquisition of P(i) as i increases until a coagulation reaction end point is detected or a blood coagulation time is calculated, after Rt equal to or smaller than the predetermined value is acquired.
[6] The method according to [5], further comprising:

acquiring Z(i) from the P(i), wherein the Z(i) is an accumulated ratio,
where

$$Z(i) = Pb(i)/Pa(i),$$

Pa(i) is a sum of P(i - m) to P(i - w),
Pb(i) is a sum of P(i + w) to P(i + m),
where i denotes the number of measurement points or time,
in a case where i indicates the number of measurement points, w = 1, and m is an integer ranging from 10 to 30,
in a case where i denotes time, w denotes a measurement time interval of the blood coagulation reaction, and m ranges from 1 to 3 seconds; and

acquiring the P(i) as the coagulation reaction end point, when the Z(i) is a predetermined threshold Zs or smaller.

[7] The method according to [6], further comprising calculating the blood coagulation time, based on the coagulation reaction end point.
[8] The method according to [5], further comprising:

acquiring C(i) from the P(i), wherein the C(i) satisfies an equation: P(C(i)) = P(i) × X%, where X ranges from 20 to 70; and

calculating the blood coagulation time, based on the acquired C(i).

[9] The method according to [5], further comprising:

calculating coagulation reaction rate data from the acquired P(i); and
calculating the blood coagulation time, based on the coagulation reaction rate data.

[10] The method according to any one of [1] to [9], further comprising repeating the step (1) until the P(i) reaches or exceeds a predetermined threshold Ps.

[11] The method according to [4], further comprising finishing the repeating of the steps and acquiring a result that the blood coagulation reaction of the blood specimen is not detected, when the i reaches a predetermined measurement end point and the Rt does not reach or is not smaller than the predetermined value.

[12] The method according to any one of [5] to [9], further comprising finishing the repeating of the steps and acquiring a result that the blood coagulation reaction of the blood specimen is not finished, when the i reaches a predetermined measurement end point and the coagulation reaction end point is not detected or the coagulation time is not calculated.

[13] The method according to any one of [5] to [9], further comprising stopping acquiring the P(i), when the coagulation reaction end point is detected or the blood coagulation time is calculated.

[14] The method according to any one of [7] to [9], further comprising outputting the calculated blood coagulation time.

[15] A program for performing the method for analyzing the blood coagulation reaction according to any one of [1] to [14].

[16] A device for performing the method for analyzing the blood coagulation reaction according to any one of [1] to [14].

Advantageous Effects of Invention

The present invention can detect an early reaction error in the measured data of the blood coagulation reaction, or distinguish the early reaction error and the true coagulation reaction from each other. The present invention can prevent adverse effects of the early reaction error on coagulation reaction analysis, for example, erroneous detection of the coagulation time. Alternatively, the present invention is applicable to a disease indicating an early reaction error, for example, DIC monitoring. The detection of an early reaction error by the present invention can be implemented in parallel with measurement of the coagulation reaction, which allows the true coagulation reaction to be analyzed in real time while measuring the coagulation reaction of the blood specimen. Consequently, in a case of analyzing a large number of specimens by the automatic analysis device, the present invention can optimize the analysis time period per specimen, and improve the entire analytical efficiency.

Brief Description of Drawings

Fig. 1 illustrates an overview of procedures for analyzing a coagulation reaction by a method of the present invention.
Fig. 2A illustrates an example of procedures for analyzing the coagulation reaction using Z(i).
Fig. 2B illustrates an example of procedures for analyzing the coagulation reaction using C(i).
Fig. 3 is a conceptual diagram illustrating a configuration of a device for implementing the method of the present invention.
Fig. 4 illustrates coagulation reaction curves of specimens of Nor, ER1, and ER2.
Fig. 5 illustrates plots of T50, T10, T5, and T100/T50, T10/T100, and T5/T100 of specimens against T100.
Fig. 6 illustrates P(i), Z(i), and i/C(i) of Nor.
Fig. 7 illustrates P(i), Z(i), and i/C(i) of ER1.
Fig. 8 illustrates P(i), Z(i), and i/C(i) of ER2.
Fig. 9 illustrates a distribution of i/C(i) when Z(i) has a predetermined value.
Fig. 10 illustrates C(i) and i/C(i) of Nor(A), ER1(B), and ER2(C).
Fig. 11 illustrates C(i) and its slopes for Nor(A), ER1(B), and ER2(C). P(i) is illustrated together in each diagram.
Fig. 12 illustrates the correlation between the coagulation time based on C(i) and the coagulation time based on Z(i).

Description of Embodiments

All Patent Literatures, Non Patent Literatures, and other publications cited in the present Description are incorporated herein by reference in their entirety.

In a blood coagulation test, a predetermined reagent is added to a blood specimen, and the subsequent blood coagulation reaction is measured. Typically, the blood coagulation reaction is represented by a coagulation reaction curve which indicates the temporal change in the amount of coagulation reaction. Conventionally, the blood coagulation time calculated based on the coagulation reaction curve is used as an indicator for detecting an error in the blood coagulation ability. In the present Description, a blood specimen, a blood coagulation reaction, and a blood coagulation time are sometimes simply called a specimen, a coagulation reaction, and a coagulation time, respectively.

Typical examples of the coagulation time include the prothrombin time (PT), activated partial thromboplastin time (APTT), thrombin time, coagulation time in fibrinogen concentration (Fbg) measurement and the like. To measure the blood coagulation reaction, typically, optical means for measuring the scattered light intensity, transmittance, absorbance or the like, or mechanical means for measuring the viscosity of plasma is used. For example, in the case of measurement based on the scattered light intensity, typically, the coagulation reaction curve exhibits a gradual increase due to the initiation of the coagulation reaction at a time point when a certain extent of time elapses after a reagent is added to a specimen, then steeply increases due to the progress of the coagulation reaction, subsequently approaches the end of the coagulation reaction and reaches a plateau, and finally exhibits a sigmoidal shape. On the other hand, a coagulation reaction curve measured based on the transmittance light intensity exhibits an inverse sigmoidal shape. Meanwhile, a coagulation reaction curve of an abnormal specimen having an abnormality in the coagulation ability exhibits various shapes depending on the causes of abnormality, such as rise time delay in and gradual increase of the coagulation reaction curve. The variability of the coagulation reaction curves of an abnormal specimen makes accurate measurement of the coagulation time by the automatic analysis device difficult.

In typical conventional blood coagulation time measurement, at least measured data until the end of the coagulation reaction is acquired, and the coagulation time is calculated based on the acquired measured data. For example, there are a method (differentiation) which determines, as the coagulation time, a time point when the coagulation reaction curve reaches the maximum rate in a period from the time point of adding the reagent to the coagulation reaction end time point after it is determined that the coagulation reaction is finished, a method (percent detection method) which determines, as the coagulation time, a time point when the amount of coagulation reaction reaches N% of that at the reaction end time point, and the like. However, an abnormal shape of the coagulation reaction curve of an abnormal specimen as described above, and after-mentioned noises cause erroneous detection of the coagulation reaction end point, for example, occurrence of detection at a too early time point, or detection of no coagulation reaction end point. As a result of such erroneous detection of the coagulation reaction end point, inaccurate coagulation time is calculated.

The measured data of the blood coagulation reaction includes various noises. These noises affect the shape of the coagulation reaction curve, and causes an impediment to accurate calculation of the coagulation time in some cases. An example thereof is a phenomenon which is increase in the coagulation reaction curve (early reaction: ER) before a time point at which a true coagulation reaction is supposed to begin, and is called an early reaction error. The early reaction error may serve as causes of causing erroneous detection of the coagulation time, and of undermining the reliability of the analysis result of the coagulation reaction. On the other hand, with a blood specimen of a disseminated intravascular coagulation (DIC) patient, an early reaction error was reported to be observed in the coagulation reaction in APTT measurement depending on the severity, and the early reaction error is expected to be used to monitor DIC (JP-A-2005-519267, and Clinical & Laboratory Haematology, 2002, 24(6): 321-327). It is desirable to detect ER included in the measured data, and avoid adverse effects on the analysis result, for the sake of accurate analysis of the coagulation reaction of the specimen. Alternatively, there is a possibility that the detected ER is useful for diagnosing and monitoring the coagulation abnormality, such as DIC.

In a case of analyzing the coagulation reactions of a large number of specimens by the automatic analysis device, it is desirable to quickly finish measurement upon detection of the end of the coagulation reaction of one specimen and then start measurement of the next specimen, in order to improve the entire analytical efficiency. However, such a method has a risk that the aforementioned erroneous detection of the end of the coagulation reaction at a too early time point erroneously terminates the measurement of a specimen undergoing the reaction in actuality, and fails to capture required measured data. On the other hand, if the measurement time per specimen is fixed to a sufficiently long time (for example, about seven minutes for APTT measurement), failure to capture measured data due to erroneous detection of the end of the coagulation reaction is prevented. However, for a large number of specimens, for example, normal specimens making up most of the specimens, such a method makes the measurement time longer than necessary. Accordingly, the entire analytical efficiency decreases.

The present invention provides a method which is for analyzing a blood coagulation reaction, detects an early reaction error included in measured data of a coagulation reaction, and allows ER and a true coagulation reaction to be distinguished from each other. Consequently, the present invention allows the coagulation reaction to be more accurately analyzed, for example, preventing erroneous detection of the end of the coagulation reaction due to ER, or accurately calculating the coagulation time. ER detection by the method for analyzing a blood coagulation reaction of the present invention can be executed in parallel with the measurement of the coagulation reaction. Consequently, this method can measure the coagulation reaction of the blood specimen while analyzing the true coagulation reaction in real time, thereby allowing the calculation of the coagulation time. This method allows the optimization of the measurement time for each specimen such that the coagulation reaction measurement with a minimum duration required to calculate each coagulation time can be performed for various specimens including normal specimens and abnormal specimens. In turn, particularly, in the case of measuring a large number of specimens by the automatic analysis device, this method can improve the analytical efficiency as a whole.

1. Method for analyzing blood coagulation reaction

**1.1** Coagulation reaction measurement

A test specimen used for the method for analyzing a blood coagulation reaction of the present invention (hereinafter, also called the method of the present invention) may be any blood specimen used for a typical blood coagulation test. Preferably, the plasma of a subject is used as the test specimen. An anticoagulant typically used for a coagulation test may be added to the test specimen. For example, blood is drawn using a blood collection tube which contains sodium citrate, and is subsequently centrifugally separated, and the plasma is obtained and is used as the test specimen.

The coagulation reaction measurement of the test specimen is performed, and the time-series data of the coagulation reaction is acquired. The coagulation reaction measurement can be executed in accordance with any method used for a blood coagulation test, for example, a method used to measure the coagulation time in a fibrinogen concentration test based on the prothrombin time (PT), activated partial thromboplastin time (APTT), or thrombin time method. To measure the coagulation reaction, general means is used; for example, optical means for measuring the scattered light intensity, transmittance, absorbance or the like, or mechanical means for measuring the viscosity of plasma is used. In the following present Description, the method of the present invention is described in accordance mainly with a coagulation reaction measurement method based on the scattered light intensity for APTT measurement. Those skilled in the art can implement application of the method of the present invention to another method for measuring the coagulation time (for example, prothrombin time (PT)).

In the measurement of the coagulation reaction, a coagulation time measurement reagent is added to the test specimen, and the coagulation reaction is initiated. The coagulation reaction in a mixture containing the reagent and the test specimen can be measured. The coagulation time measurement reagent to be used can be selected in conformity with the measurement purpose. Reagents for measuring various coagulation times are commercially available (for example, APTT reagent Coagpia APTT-N manufactured by Sekisui Medical Co., Ltd.).

The reaction initiation time point of the coagulation reaction can typically be defined as a time point at which calcium ion (for example, calcium chloride solution) is added and the coagulation reaction is initiated. Alternatively, another timing may be defined as a reaction initiation time point. The duration of measuring the coagulation reaction can approximately range, for example, from several tens of seconds to seven minutes from the time point of mixing the specimen and the reagent. The measurement time may be a freely defined fixed value, or may be up to a time point at which the end of the coagulation reaction of each specimen is detected. During the measurement time, the measurement of the progress of the coagulation reaction (photometry in the case of optical detection) can be repeatedly performed at predetermined intervals. For example, measurement may be performed at intervals of 0.1 seconds. The temperature of the mixture during the measurement is under a typical condition, for example, between 30°C and 40°C, inclusive, preferably, between 35°C and 39°C, inclusive. Various conditions of the measurement can be set in conformity with the specimen, reagent, measurement means and the like as appropriate.

A series of operations in the coagulation reaction measurement described above can be performed using an automatic analysis device. An example of the automatic analysis device may be a blood coagulation automatic analysis device CP3000 (manufactured by Sekisui Medical Co., Ltd). Alternatively, some operations may be manually performed. For example, preparation of the test specimen can be performed by a person, and operations thereafter can be performed by the automatic analysis device.

1.2 Acquisition of coagulation reaction P

The method of the present invention sequentially acquires the coagulation reaction $P(i)$ from the reaction initiation time point. Here, "i" denotes the number of measurement points or time from the coagulation reaction initiation (also simply called the number of measurement points, or time, respectively). For example, in the case where a measurement (photometry) interval is 0.1 seconds, it is represented that time = $0.1 \times$ the number of measurement points. That is, $P(i)$ is a function of a variable i, and may be a function of the number of measurement points or a function of time. In the following present Description, $P(i)$ is sometimes simply abbreviated as P.

Typically, the coagulation reaction P is obtained by applying a denoising or a smoothing process to measurements of the coagulation reaction measurement (for example, photometric measurements of the scattered light intensity) by typical means, or applying zero adjustment for adjusting the initial value or curve relativizing as required. Any of various publicly known methods related to denoising can be used for the smoothing process for the measurements. For example, the smoothing process may be a filtering process, a process of obtaining the derivative by the computation of a difference or an after-mentioned intra-interval average slope and subsequently integrating it, or the like. In the zero adjustment, for example, the smoothed measurement may be adjusted so as to set the value at the measurement initiation time point to zero. Preferably, the measurements are smoothed and zero-adjusted, and the coagulation reaction P(i) is acquired.

1.3 Analysis of coagulation reaction based on Rt

From the P(i), t1 and t2 are acquired. Both t1 and t2 represent the number of measurement points or time, and t2 < t1. t1 may be any point selected from the numbers of measurement points or time at which P(i) of the specimen is previously acquired, and t2 satisfies the following equation (1).

$$P(t2) = P(t1) \times X\% \quad \dots \quad (1)$$

These P(t1) and P(t2) represent the coagulation reaction P at t1 and t2, respectively. X ranges from 20 to 70 and, preferably, ranges from 40 to 60.

Based on the acquired ratio (t1/t2) of t1 to t2, it can be distinguished whether the coagulation reaction P(t1) at t1 is the early reaction (ER) or the true coagulation reaction. Hereinafter, t1/t2 is also represented as Rt.

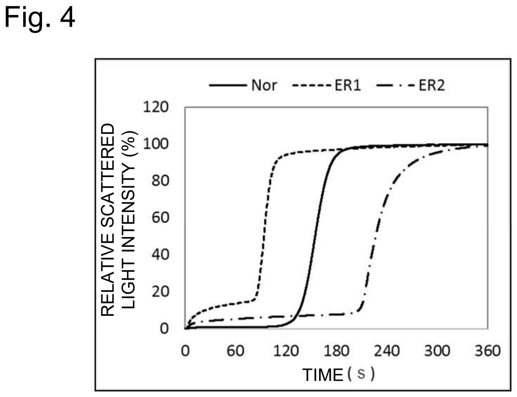

As described in after-mentioned Examples, when ER occurs, the coagulation reaction curve exhibits a small rise very early in the measurement and then gradually increases, and subsequently, a true coagulation reaction begins, thus increasing the curve steeply. As a result, the coagulation reaction curve increases in two stages (see Fig. 4). The increase of the coagulation reaction curve caused by ER before increase due to such a true coagulation reaction begins causes erroneous detection of the coagulation reaction end, and serves as a cause of inaccurate coagulation time. In detail, in the conventional coagulation time measurement, a method of determining, as the coagulation time, a time point at which the amount of coagulation reaction reaches N% of the reaction end time point (what is called a percent detection method) is often used. On the other hand, the method of detecting the coagulation reaction end point may be any of a method of adopting a time point at which the coagulation reaction curve reaches a plateau, as the end of the coagulation reaction, a method of adopting a time point at which the accumulated ratio Z falls below a threshold, as the end of the coagulation reaction, a method of using a peak of the coagulation reaction rate curve as a reference, and the like. However, in accordance with any method, ER may be erroneously recognized as a true coagulation reaction, and the end of the coagulation reaction may be detected midway through the early reaction in some cases. As a result, erroneous coagulation time is sometimes calculated.

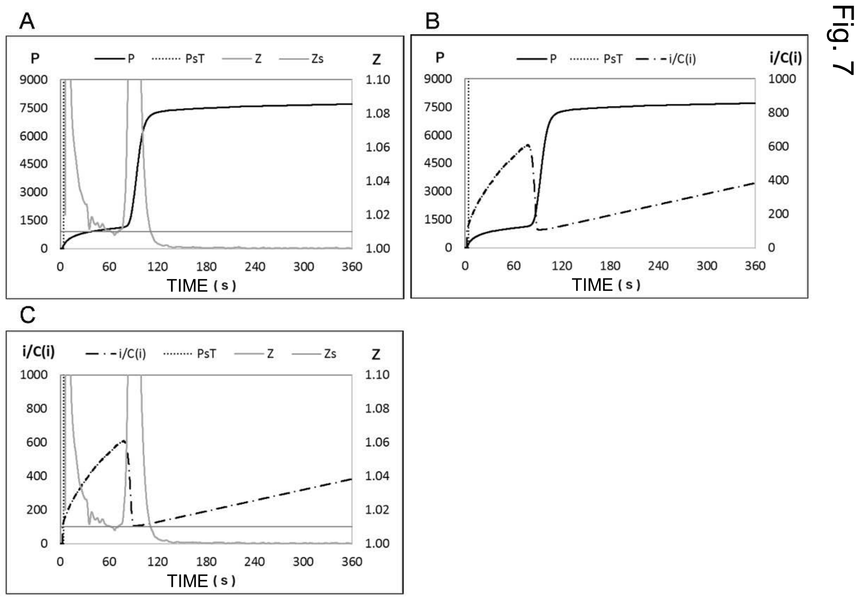

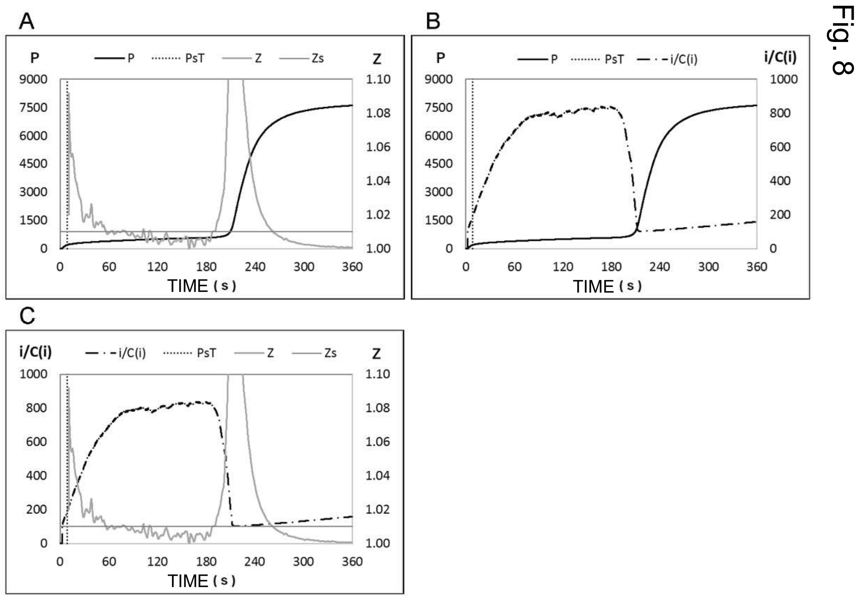

The Rt indicates a high value in which the gradual increase of the reaction curve immediately after the measurement begins is reflected, during ER, and subsequently, steeply decreases at the rise time point of the true coagulation reaction (see Figs. 7B and 8B). Consequently, ER can be detected with reference to Rt. Specifically, the coagulation reaction P(t1) at the t1 is evaluated as ER if Rt is higher than a predetermined value S, and as the true coagulation reaction if it is not. The predetermined value S can be set as appropriate in consideration of the difference in measured data of the coagulation reaction between a specimen where ER appears and a specimen where it does not appear, and ranges, for example, from 150 to 240.

1.4 Acquisition of Rt in real time and coagulation reaction analysis

In parallel with the coagulation reaction measurement, the coagulation reaction P(i) of the specimen, and Rt based thereon are sequentially acquired, which allows detection of ER in real time, and correct analysis of the coagulation reaction (for example, calculation of the coagulation time) based on the true coagulation reaction, while performing the coagulation reaction measurement. For example, when P(i) at any i is obtained as i increases (progress of measurement), it is assumed that t1 = i, and t2 is obtained in accordance with the equation (1). Preferably, a threshold Ps (described later) for detecting the initiation of the reaction is set, and when P(i) at any i after P(i) > Ps holds is obtained, it is assumed that t1 = i, and t2 is obtained in accordance with the equation (1). Next, Rt (= t1/t2) at t1 = i is calculated. The t2 and Rt are represented as functions of the variable i. Accordingly, they can be defined as C(i) and Rt(i) That is, C(i) and Rt(i) satisfy the following equations (1)' and (2).

$$P(C(i)) = P(i) \times X\% \quad \dots (1)'$$

$$Rt(i) = i/C(i) \quad \dots (2)$$

(X is as defined above.)

Based on the Rt(i), it can be evaluated whether the P(i) is the true blood coagulation reaction or not. Specifically, if the Rt(i) is higher than the predetermined value S, the P(i) is ER. Accordingly, the measurement of the coagulation reaction is continued until the true coagulation reaction is obtained. That is, until the Rt(i) reaches or becomes smaller than the predetermined value S, it is assumed that i = i + k, and acquisition of new P(i), t1, t2, and Rt(i) is repeated (where, k > 0, and for example, in the case where i indicates the number of measurement points, it is preferable that k = 1 to 10, and in the case where i indicates time, it is preferable that k = measurement time interval (second) × 1 to 10).

If it is confirmed that the Rt(i) reaches or is smaller than the predetermined value S, a subsequently acquired P(i) can be assumed as the true coagulation reaction. Here, that is, t1 at which the Rt(i) reaches the predetermined value S or a smaller value is assumed as Tr. P(i) which is acquired after Tr and assumed as the true coagulation reaction can be used to analyze the coagulation reaction. Consequently, the measurement of the coagulation reaction is continued even after the Tr, and new P(i) and the coagulation reaction curve can be acquired. The data can be used for the analysis of the coagulation reaction, for example, the detection of the coagulation reaction end point, the calculation of the coagulation time and the like. That is, in accordance with the present invention, the coagulation reaction end point and the coagulation time of the test specimen are detected in a period after the Tr. Specifically, i is increased until the coagulation reaction end point is detected or the coagulation time is calculated after the Tr. That is, it is assumed that i = i + k (k = 1 or the measurement time interval), and the measurement of the coagulation reaction and acquisition of P(i) are repeated. Here, at the same time or regular intervals, the acquisition of t1, t2, and Rt(i) may be repeated. However, there is no need to do so. After the coagulation reaction end point or the coagulation time is detected, the measurement of the coagulation reaction and the acquisition of the new P(i) may be finished, or may continue instead.

1.5 Off-line acquisition of Rt and coagulation reaction analysis

After the coagulation reaction measurement is finished, Rt(i) or C(i) can be calculated in accordance with the equations (1)' and (2) using the obtained P(i). If Rt(i) is higher than the predetermined value S, P(i) indicates ER. On the other hand, if i when Rt(i) reaches or is less than the predetermined value S is assumed as Tr, P(i) after the Tr is the true coagulation reaction. P(i) assumed as the true coagulation reaction can be used for the analysis of the coagulation reaction, for example, the detection of the coagulation reaction end point, the calculation of the coagulation time and the like.

1.6 Other description about Rt

In the following present Description, Rt(i) is sometimes abbreviated as Rt. Consequently, in the following present Description, "Rt" comprehensively indicates Rt at a specific t1 and Rt(i) at i. It can be determined which meaning is indicated based on the context. Basically, in the cases where the real-time analysis as in the above 1.4, and the off-line analysis as in the above 1.5 are performed, "Rt" means Rt(i) at i.

In the present invention, instead of Rt (= t1/t2 or i/C(i)), its reciprocal 1/Rt (= t2/t1 or C(i)/i) can be used as the reference for detecting ER. Specifically, it is determined to be ER if 1/Rt is smaller than a predetermined value S', and to be a true coagulation reaction if it is not. The predetermined value S' can be set as appropriate, and, for example, ranges from 1/150 to 1/240.

1.7 Threshold Ps

Immediately after the measurement of the coagulation reaction begins, t1 and t2 are close to each other, and Rt is lower than the predetermined value S accordingly. To prevent erroneous detection of ER or the true coagulation reaction due to a small interval between t1 and t2 immediately after the measurement begins, it is preferable not to acquire Rt or compare Rt with the predetermined value S in a certain time period after the measurement begins. Alternatively, it is preferable not to acquire Rt or compare Rt with the predetermined value S until P(i) reaches or exceeds the threshold Ps (that is, the step of acquiring Rt is skipped, and new P(i) is acquired).

On the other hand, even with a specimen substantially having no ER, Rt sometimes has a high value by a slight change of the coagulation reaction curve before a main reaction. In such a case, Rt is not acquired or Rt is not compared with the predetermined value S in a certain time period after the measurement begins, or Rt is not acquired or Rt is not compared with the predetermined value S until P(i) reaches or exceeds the threshold Ps, which can prevent ER from being erroneously detected.

2. Acquisition of coagulation reaction end point and coagulation time

In the present invention, the accumulated ratio Z(i) or the coagulation reaction rate data V(i) may be acquired from P(i) acquired as described above. The Z(i) and V(i) can be used to detect the coagulation reaction end point of the test specimen or calculate the coagulation time.

2.1 Accumulated ratio Z(i)

The accumulated ratio Z(i) represents the integrated value of P(i) in a minute time period, and is calculated by the following equation (3): Z(i) = Pb(i)/Pa(i) ... (3) where

Pa(i) is the sum of P(i - m) to P(i - w),

Pb(i) is the sum of P(i + w) to P(i + m),

i denotes the number of measurement points or time, and

In the case where i indicates the number of measurement points, w = 1, and in the case where i indicates time, w denotes the measurement time interval for the coagulation reaction.

That is, in the case where i indicates the number of measurement points, Z(i) can also be represented by the following equation (3)'.

$$Z(i) = \{P(i + 1) + P(i + 2) + ... + P(i + m)\}/\{P(i - m) + P(i - m + 1) + ... + P(i - 1)\} \tag{3`}$$

m can be set as appropriate depending on the measurement condition of the coagulation reaction, the analysis item and the like. Preferably, the duration from i - m to i - w (or from i + w to i + m) is set to range from 1 to 3 seconds. For example, in the case where i indicates the number of measurement points, m indicates (1 to 3 seconds)/measurement time interval (seconds), for example, an integer ranging from 10 to 30. That is, in a case where the measurement time interval is 0.1 seconds, it is preferable that m range from 10 to 30, and in a case where the measurement time interval is 0.2 seconds, it is preferable that m range from 5 to 15. The optimal value of m may be selected so as to allow the coagulation time to be appropriately calculated in consideration of the measurement condition. In the following present Description, Z(i) is sometimes simply abbreviated as Z.

The coagulation reaction P(i) at the earliest measurement point or time when Z(i) reaches or becomes less than the threshold Zs is detected as the coagulation reaction end point Pe. Zs can be set as appropriate depending on the analysis item. For example, in the case of APTT measurement, it is preferable that Zs be less than or equal to 1.050, more preferably, set in a range from 1.010 to 1.001. To prevent erroneous detection of Pe due to ER, it is preferable that Z(i) be calculated after the Tr. Preferably, Z(i) is calculated after P(i) reaches or exceeds the threshold Ps.

More preferably, P(i) is sequentially acquired, while the coagulation reaction is measured (as i increases). After P(i) reaches or exceeds the threshold Ps, Rt is acquired in real time based on the P(i) sequentially acquired as described above, and is compared with the predetermined value S. After the Rt reaches the predetermined value S or a value smaller than this value (that is, after Tr), Z(i) is sequentially acquired based on the sequentially acquired P(i). P(i) when Z(i) reaches or falls below the predetermined threshold Zs for the first time is detected as the coagulation reaction end point Pe.

Alternatively, P(i) is sequentially acquired, Z(i) is then sequentially acquired after the acquired P(i) reaches or exceeds the threshold Ps, and if Z(i) reaches the predetermined threshold Zs or less and Rt based on the corresponding P(i) reaches the predetermined value S or a value smaller than this value, the P(i) is detected as the coagulation reaction end point Pe.

In this procedure, while the coagulation reaction is measured, P(i) and Z(i) are acquired in real time, and Pe can be detected. After Pe is detected, the measurement of the coagulation reaction and the acquisition of the new P(i) may be finished, or may continue instead.

2.2 Coagulation reaction rate data V(i)

The coagulation reaction rate data V(i) is obtained by taking the first derivative of P(i). The differential process can be executed by any method, and can be performed by, for example, calculating the intra-interval average slope. In the intra-interval average slope calculation, a predetermined number of measurement points before and after each measurement point i, for example, 2K + 1 measurement points from i - K to i + K can be used. For example, in the case where i indicates the number of measurement points, total five (i - 2)-, (i - 1)-, i-, (i + 1)-, and (i + 2)-th measurement points can be used. The average slope means a slope when these measurement points are linearly approximated. A usual method, such as a least-squares method, can be used as the computation method for the linear approximation. The average slope of these measurement points can be assumed as the first derivative at the measurement point i.

Based on V(i), the coagulation reaction end point Pe can be detected. For example, after the peak of V(i) reaches the maximum, P(i) at the earliest number of measurement points or time at which S% of the maximum or less is reached can be detected as the coagulation reaction end point Pe (see Patent Literature 5).

More preferably, P(i) is sequentially acquired while the coagulation reaction is measured (as i increases), and after P(i) reaches or exceeds the threshold Ps, V(i) is calculated based on the sequentially acquired P(i), and Pe is detected based on the V(i). After Pe is detected, the measurement of the coagulation reaction and the acquisition of the new P(i) may be finished, or may continue instead.

2.3 C(i)

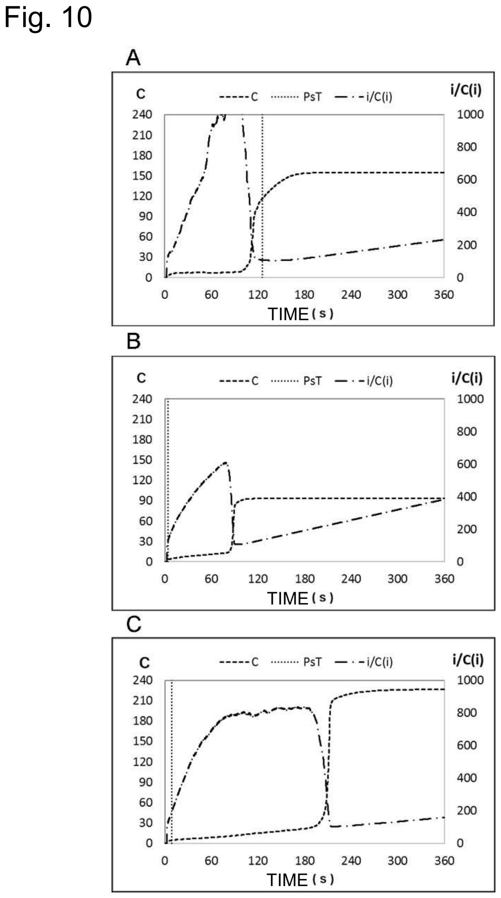

In the case where it is assumed that t1 = i, and t2 is sequentially acquired from P(i) as described in the above 1.4, t2 is represented as a function of the variable i, and the function is defined as C(i). The coagulation time can be calculated based on the C(i). Specifically, as described in after-mentioned Examples, with steep increase in P(i) due to the true coagulation reaction, C(i) steeply increases, and subsequently, the increase becomes gradual as the coagulation reaction approaches the end, and a plateau is reached (see Fig. 10). Consequently, with C(i) as an indicator, the coagulation time can be calculated. For example, the time when the change rate of C(i) satisfies a predetermined condition can be regarded as the coagulation time. For example, the change rate of C(i) can be obtained as the average amount of change of C(i) in a predetermined range (for example, in the case where i is the number of measurement points, $[\{C(i) - C(i - k)\}/k]$; it is preferable that k range from 5 to 20), and the time when the average amount of change reaches the threshold (for example, from 0.06 to 0.01) or less can be regarded as the coagulation time. For example, the change rate of C(i) can be obtained as the slope of the tangent of C(i) (for example, the intra-interval average slope of total 2k + 1 items of C from C(i - k)- to C(i + k)-th; in the case where i indicates the number of measurement points, it is preferable that k range from 5 to 20), and the time when the slope of the tangent reaches the threshold (for example, from 0.01 to 0.06) or less can be assumed as the coagulation time. In the following present Description, C(i) is sometimes simply abbreviated as C.

More preferably, P(i) is sequentially acquired while the coagulation reaction is measured (as i increases), and after P(i) reaches or exceeds the threshold Ps, C(i) is calculated based on the sequentially acquired P(i) as described above, and the average amount of change or the intra-interval average slope is compared with the threshold Cs. The time point at which the average amount of change or the intra-interval average slope reaches the threshold Cs or a smaller value for the first time is calculated as the coagulation time. After the coagulation time is calculated, the measurement of the coagulation reaction and the acquisition of new P(i) may be finished, or may continue instead.

2.4 Calculation of coagulation time

The coagulation time can be calculated based on the Z(i), V(i), or C(i). In one example, based on the coagulation reaction end point Pe acquired using the Z(i) or V(i) as described above, the coagulation time can be calculated. For example, the time point at which P(i) reaches N% of the coagulation reaction end point Pe can be calculated as the coagulation time (percent detection method). Other examples of the method of calculating the coagulation time include: a method of assuming, as the coagulation time, the time when the change rate of C(i) (for example, the average amount of change or intra-interval average slope of C(i) described above) reaches the threshold or less (the above 2.3); a method of calculating, as the coagulation time, the time point at which the peak of V(i) reaches the maximum (differentiation); and a method of calculating the coagulation time, based on the temporal change in Z(i) (see Patent Literature 4).

In parallel with the coagulation reaction measurement (so-called in real time), the coagulation time can be calculated using the Z(i), V(i), or C(i). However, the calculation of the coagulation time using the Z(i) V(i), or C(i) may be performed after the coagulation reaction measurement is finished. Alternatively, after the coagulation reaction measurement is finished, the true coagulation reaction can be analyzed in accordance with a conventional typical procedure, and the coagulation time can be calculated. For example, the time point when the true coagulation reaction P(i) is at the maximum can be detected as the coagulation reaction end point Pe, and the time point at which P(i) reaches N% of Pe can be determined as the coagulation time (percent detection method).

3. Output of analysis result

The method of the present invention may encompass outputting a result obtained by the analysis. The analysis result can include information, such as presence or absence of ER, data of the detected ER or the true coagulation reaction (reaction curve and the like), the coagulation reaction end point, and the coagulation time.

Test specimens with abnormal coagulation reactions sometimes include a specimen which has a too small coagulation reaction and in which the detection of the reaction is difficult, a specimen the initiation of the coagulation reaction of which is unclear and the coagulation reaction curve of which does not steeply rise, and a specimen time to the end of the coagulation reaction of which is long or the end of the coagulation reaction of which is unclear. With such specimens, in accordance with the method of the present invention, even if the coagulation reaction measurement continues for a predetermined time period, errors sometimes occur in which P(i) does not reach Ps and the Rt cannot be acquired, the Rt does not reach the predetermined value S, or even if the Rt reaches the predetermined value S, the coagulation reaction end point Pe cannot be detected. In such cases, the method of the present invention can output information about these errors, for example, occurrence of errors or error types.

In one example, if i reaches the predetermined maximum measurement time (measurement end point), and Rt does not reach or is not smaller than the predetermined value S, the repeating of steps of acquiring the P(i), t1, t2, and Rt is finished. In this case, a result that no true coagulation reaction of the test specimen has been detected is acquired.

In another example, if i reaches the predetermined maximum measurement time (measurement end point), and the coagulation reaction end point Pe is not detected or the coagulation time is not calculated, the repeating of steps of acquiring the P(i), t1, t2, and Rt is finished. In this case, a result that the coagulation reaction of the test specimen has not been finished is acquired.

4. Other applications

The method for analyzing a blood coagulation reaction by the present invention is performed basically using the coagulation reaction measured based on the scattered light intensity. However, those skilled in the art can implement applications using other means for the method of the present invention, for example, using the coagulation reaction measured based on the transmittance, absorbance or the like.

As for the detection reference in the present Description, those skilled in the art can understand that as long as an intended coagulation reaction or ER can be detected, "greater than or equal to" the threshold or the predetermined value, and "greater than" the threshold or the predetermined value can be interchangeably used, and likewise, "less than or equal to" the threshold or the predetermined value, and "less than" the threshold or the predetermined value can be interchangeably used.

5. Exemplary analysis procedure

As an example of procedures for analyzing the coagulation reaction by the method of the present invention, the procedures for measuring the coagulation time are described below. The procedures described below are an example, and can be subjected to normal modification by the those skilled in the art, for example, change of the order of computation.

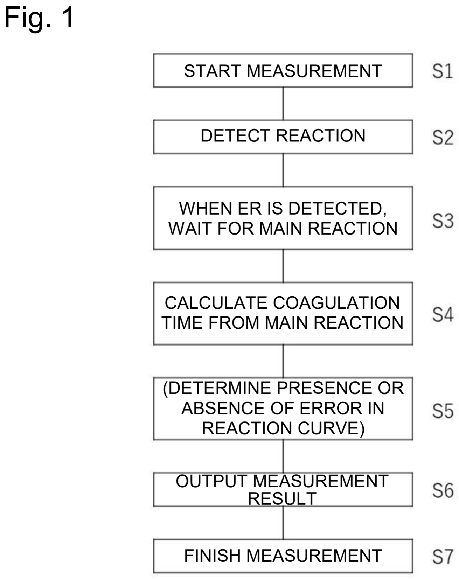

An overview of the coagulation time measurement procedures is described below with reference to Fig. 1.

S1: Start measurement of the coagulation reaction.
S2: When P(i) > Ps, it is determined that a reaction is present.
S3: When ER is detected based on Rt, wait for a true coagulation reaction (main reaction).
S4: Calculate the coagulation time from the main reaction.
S5: (Optional) Determine presence or absence of an error in the coagulation reaction.
S6: Output the coagulation time. (Optional) In conjunction therewith, output information about detection of ER or an error in the coagulation reaction.
S7: Finish the measurement. When the coagulation time is calculated, the measurement is finished, or it is finished at a predetermined time.

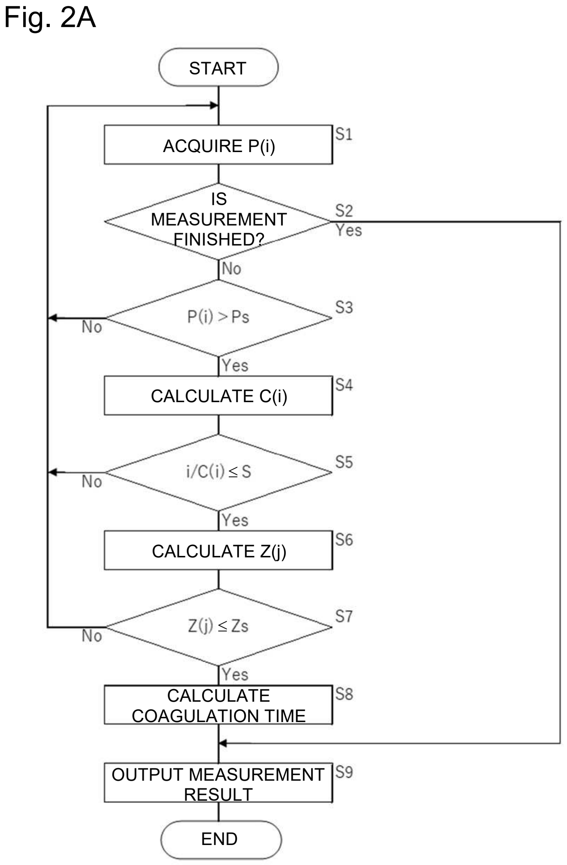

Furthermore, an example of the coagulation time measurement procedures using Z(i) is described below with reference to Fig. 2A.

S1: Acquire P(i).
S2: When the maximum measurement time (measurement end point) is exceeded, the measurement is finished. (Optional) Record the detection of an error in the coagulation reaction.
S3: Until P(i) > Ps holds, the processing returns to S1, and the acquisition of P(i) continues.
S4: Calculate C(i) satisfying $P(C(i)) = P(i) \times X\%$.
S5: Until $i/C(i) \leq S$ holds, the processing returns to S1, and the acquisition of P(i) continues. (Optional) When $i/C(i) > S$ holds, record ER detection.
S6: Calculate Z(j) from 2m + 1 items of P from P(i - 2m) to P(i). (j = i - m)
S7: Until $Z(j) \leq Zs$ holds, the processing returns to S1, and the acquisition of P(i) continues.
S8: Assume P(j) as the coagulation reaction end point, and calculate the coagulation time.
S9: Output the coagulation time calculated in S8.

(Optional) If Yes in S2, output that no coagulation time was obtained.

(Optional) In conjunction therewith, output information about detection of ER or an error in the coagulation reaction.

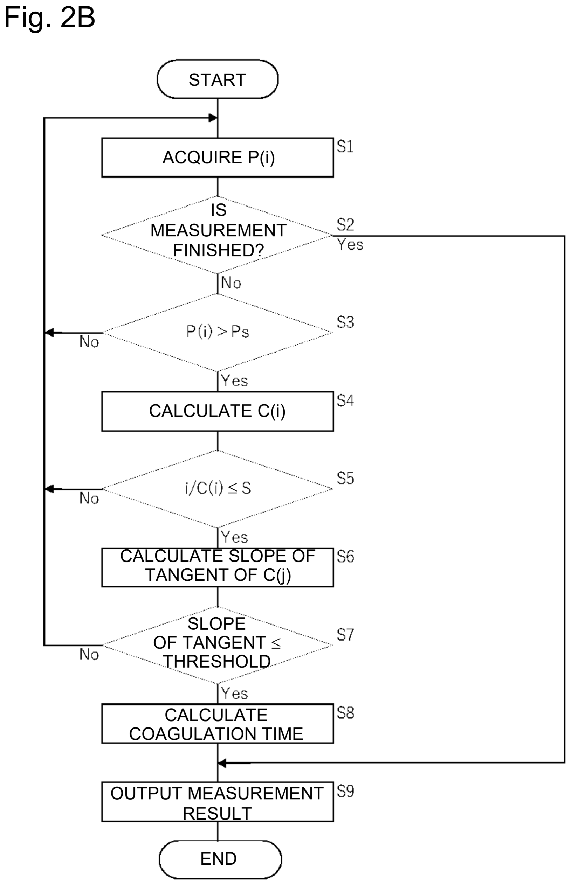

Furthermore, an example of the coagulation time measurement procedures using C(i) is described below with reference to Fig. 2B.

S1: Acquire P(i).
S2: When the maximum measurement time (measurement end point) is exceeded, the measurement is finished. (Optional) Record the detection of an error in the coagulation reaction.
S3: Until P(i) > Ps holds, the processing returns to S1, and the acquisition of P(i) continues.
S4: Calculate C(i) satisfying $P(C(i)) = P(i) \times X\%$.
S5: Until $i/C(i) \leq S$ holds, the processing returns to S1, and the acquisition of P(i) continues. (Optional) When $i/C(i) > S$ holds, record ER detection.
S6: Calculate the slope of the tangent of C(j) from 2k + 1 items of C from C(i - 2k) to C(i). (j = i - k)
S7: Until the slope of tangent of C(j) $\leq$ threshold holds, the processing returns to S1, and the acquisition of P(i) continues.
S8: Assume C(j) as the coagulation time.
S9: Output the coagulation time calculated in S8.

(Optional) If Yes in S2, output that no coagulation time was obtained.
(Optional) In conjunction therewith, output information about detection of ER or an error in the coagulation reaction.

6. Program and device

The method for analyzing the blood coagulation reaction of the present invention can be automatically performed using a computer program. Consequently, an aspect of the present invention is a program for performing the aforementioned method for analyzing the blood coagulation reaction of the present invention. A series of steps of the aforementioned method of the present invention can be automatically performed by an automatic analysis device. Thus, an aspect of the present invention is a device for performing the aforementioned method for analyzing the blood coagulation reaction of the present invention. Execution of the method of the present invention by the device can be controlled by the program of the present invention. Another aspect of the present invention is a system for performing the method for analyzing the blood coagulation reaction of the present invention. The system includes the device or the program for performing the aforementioned method for analyzing the blood coagulation reaction of the present invention.

The program of the present invention controls, for example, execution of the procedures illustrated in Fig. 2A or 2B.

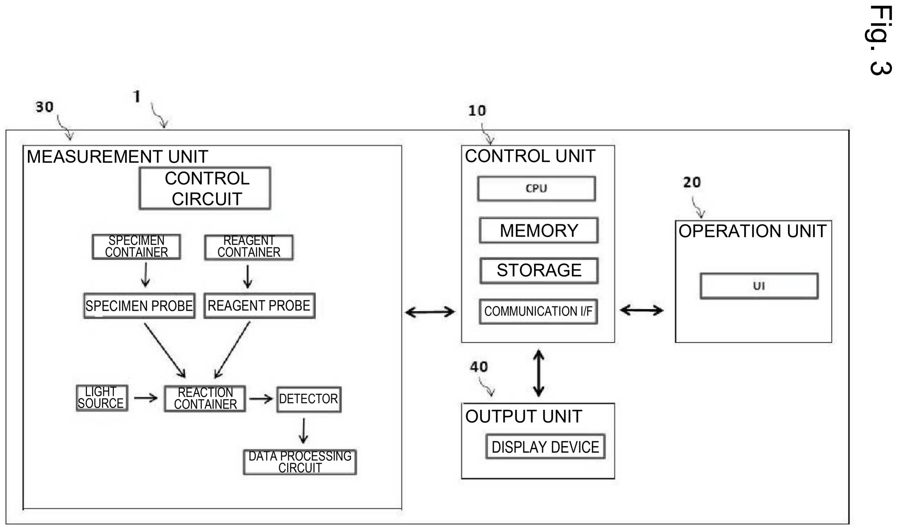

One embodiment of the device of the present invention is described below. One embodiment of the device of the present invention is an automatic analysis device 1 as illustrated in Fig. 3. The automatic analysis device 1 includes a control unit 10, an operation unit 20, a measurement unit 30, and an output unit 40.

The control unit 10 controls the entire operation of the automatic analysis device 1. The control unit 10 may be made up of a computer (for example, a personal computer). The control unit 10 includes a CPU, a memory, a storage, a communication interface (I/F) and the like, and can perform processing of commands from the operation unit 20, control of the operation of the measurement unit 30, storing and data analysis of measured data received from the measurement unit 30, storing of an analysis result, control of outputting of the analysis result by the output unit 40 and the like. Furthermore, the control unit 10 may be connected to other devices, such as an external medium, and a host computer. Note that in the control unit 10, the computer which controls the operation of the measurement unit 30, and the computer which analyzes data measured by the unit 30 may be identical to or different from each other.

The operation unit 20 acquires input from an operator, and transmits the obtained input information to the control unit 10. For example, the operation unit 20 includes a user interface (UI), such as a keyboard and a touch panel. Under control by the control unit 10, the output unit 40 outputs the analysis result of the coagulation reaction, and as required, the coagulation reaction data measured by the measurement unit 30, and analysis results based thereon, such as P(i), Rt, Z(i), V(i), C(i), Pe, the coagulation time, ER detection, and errors. For example, the output unit 40 includes a display device, such as a display.

The measurement unit 30 executes the series of operations for the blood coagulation test, and acquires measured data of the coagulation reaction of a sample which contains the blood specimen. The measurement unit 30 includes various instruments and analysis modules required for a blood coagulation test, for example, a specimen container which stores the blood specimen, a reagent container which stores a reagent for the test, a reaction container for the reaction between the specimen and the reagent, a probe for dispensing the blood specimen and the reagent into the reaction container, a light source, a detector for detecting scattered light or transmitted light from the sample in the reaction container, a data processing circuit which transmits data from the detector to the control unit 10, and a control circuit which controls the operation of the measurement unit 30 in response to an instruction of the control unit 10.

The control unit 10 performs analysis of the coagulation reaction of the specimen, based on the coagulation reaction data. This analysis can include the aforementioned acquisition of P(i), Rt, Z(i), V(i), and C(i), detection of ER and Pe, and calculation of the coagulation time. Alternatively, this analysis may be executed by the control unit 10 based on the measured data from the measurement unit 30, or may be executed by another device, for example, the measurement unit 30, and the result may be transmitted to the control unit 10. The control unit 10 may store thresholds used to detect ER and Pe, or the control unit 10 may import the thresholds stored in an external device or on a network, for the sake of the detection.

This analysis described above can be executed by the program for performing the method of the present invention. Consequently, the control unit 10 may include the program for performing the method for analyzing the blood coagulation reaction of the present invention.

Examples

The present invention is described in further detail below with reference to Examples. However, the present invention is not limited to these Examples.

1. Method

1) Sample

Total 1,062 specimens which include plasma with a coagulation abnormality, and plasma with no coagulation abnormality were used as test specimens.

2) Reagent

Coagpia APTT-N (manufactured by Sekisui Medical Co., Ltd.) was used as the reagent for APTT measurement. Coagpia APTT-N calcium chloride solution (manufactured by Sekisui Medical Co., Ltd.) was used as the calcium chloride solution.

3) Coagulation reaction measurement

The measurement of the coagulation reaction in a sample was performed using a blood coagulation automatic analysis device CP3000 (manufactured by Sekisui Medical Co., Ltd.). 50$\mu$L of the specimen was heated in a cuvette at 37°C for 45 seconds, 50$\mu$L of the reagent for APTT measurement at approximately 37°C was subsequently added, and after 171 seconds elapsed, 50$\mu$L of the calcium chloride solution was added, thus initiating the coagulation reaction. The reaction was performed at 37°C. In the measurement of the coagulation reaction, the cuvette was irradiated with light of a wavelength of 660 nm from a light source which is an LED, and the scattered light intensity of 90-degree side scattered light was measured at 0.1-second intervals. The measurement time was 360 seconds.

4) Creation of coagulation reaction curve

To the photometric measured data, a smoothing process including denoising was applied and then a zero adjustment process was applied so as to allow the scattered light intensity at the photometry initiation time point to be zero, and the coagulation reaction curve P(i) was created. The maximum of the reaction curve is assumed as Pmax.

5) Acquisition of accumulated ratio Z

The accumulated ratio Z(i) of the coagulation reaction curve P(i) (see Patent Literature 4) was calculated as follows.

$$\texttt{Accumulated ratio Z(i) = Pb(i)/Pa(i)}$$

Pa(i) = sum of P(i - 20) to P(i - 1)

Pb(i) = sum of P(i + 1) to P(i + 20)

(To avoid ER,) the earliest time point at which Z(i) becomes less than the threshold Zs (= 1.010) after P(i) reaches 50% of Pmax was assumed as the coagulation reaction end time point T100 (seconds), and T50, T10, and T5 (seconds) respectively satisfying the following equations were obtained.

$$P(T50) = P(T100) \times 50\%$$

$$P(T10) = P(T100) \times 10\%$$

$$P(T5) = P(T100) \times 5\%$$

6) Classification of specimens

Based on the shape of P(i), test specimens (n = 1,062) were classified into three groups.

Nor: without early reaction (ER) (n = 1,043)
ER1: with ER having a magnitude of 10% of P(T100) or higher (n = 5)
ER2: with ER having a magnitude of 5% of P(T100) or higher from which ER1 was excluded (n = 14)

7) Acquisition of C(i)

It was assumed that t1 = i, C(i) satisfying the following equation was calculated.

$$P(C(i)) = P(i) \times X\% \quad (X = 50)$$

i/C(i) was obtained.

2. Results

1) Coagulation reaction curve

Fig. 4 illustrates examples of coagulation reaction curves of specimens of Nor (solid line), ER1 (dotted line), and ER2(chain line). The coagulation reaction curves illustrated in Fig. 4 represent the relative scattered light intensity, with Pmax of each specimen being assumed as 100%. With Nor, no change of the reaction curve was exhibited until the main reaction appeared. On the other hand, with ER1 and ER2, the reaction curve rose immediately after the initiation of the measurement due to ER, continued to increase gradually, and subsequently exhibited a steep increase due to the main reaction. The magnitude of the ER portion exceeded 10% for ER1 and 5% for ER2 with respect to Pmax.

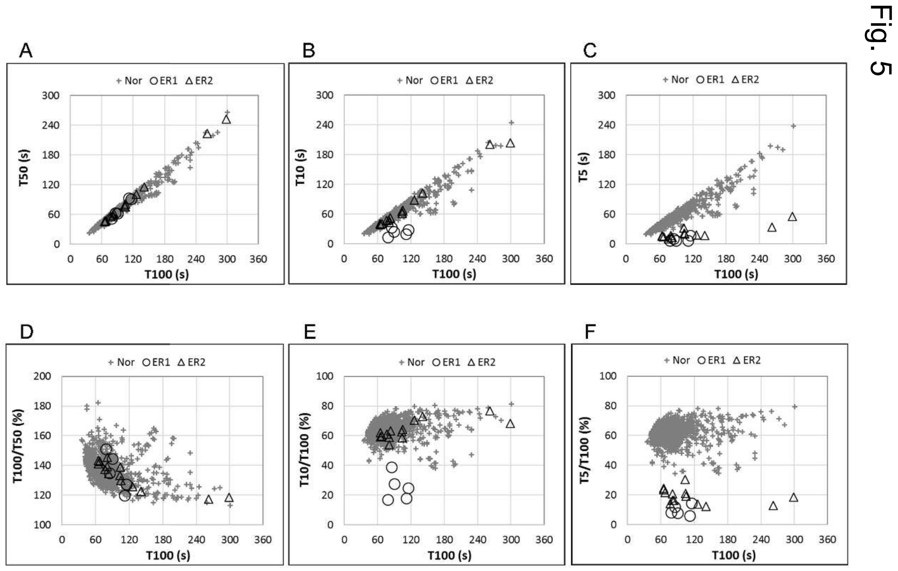

Figs. 5A, 5B, and 5C illustrate plots of T50, T10, and T5 of each specimen with respect to T100, and Figs. 5D, 5E, and 5F illustrate plots of T100/T50, T10/T100, and T5/T100. Distributions of the groups of Nor(+), ER1(○), and ER2(△) overlap for T50 (Figs. 5A and 5D). For T10 (Figs. 5B and 5E), ER1 positions are located below. For T5 (Figs. 5C and 5F), both ER1 and ER2 are located below Nor. Based on these results as well, it was confirmed that for ER1, ER with the magnitude of 10% of P(T100) or higher appeared earlier than the main reaction, and for ER2, ER with the intensity of 5% of P(T100) or higher appeared earlier than the main reaction.

2) Z(i) and i/C(i)

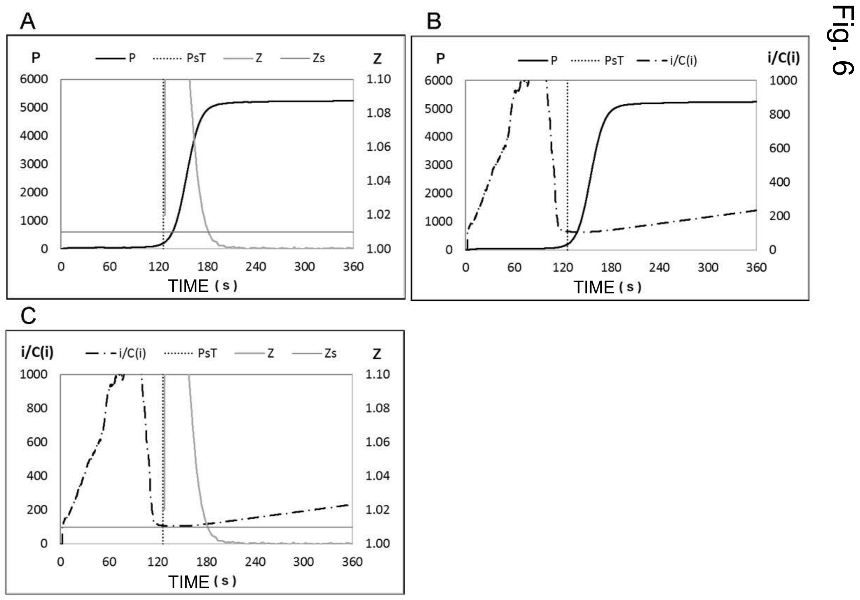

Figs. 6 to 8 illustrate examples of P(i), Z(i), and i/C(i) for Nor, ER1, and ER2, respectively. In Figs. 6 to 8, P(i) and Z(i) are represented respectively as P and Z. In Figs. 6A and 6B to 8A and 8B, Z(i) and i/C(i) are illustrated with P(i). In Figs. 6C to 8C, Z(i) is illustrated with i/C(i). In Figs. 6 to 8, the dotted line along the Y-axis of each diagram indicates a time point PsT at which P(i) exceeds the threshold Ps (= 200), and the dotted line along the X-axis of A indicates Zs. As illustrated in Figs. 6A to 8A, for Nor, Z(i) ≤ Zs did not hold before the main reaction rose. Meanwhile, for ER1 and ER2, Z(i) ≤ Zs held before the main reaction. This indicates that for ER1 and ER2, the coagulation reaction end time point T100 was detected at the ER portion as long as there was no other constraint condition, and an erroneous coagulation time was calculated.
As illustrated in Figs. 6B to 8B, for all the three groups, i/C(i) had a high value and allowed a peak to appear before the main reaction, and linearly increased after the main reaction began. The observation of the positional relationship between the peak of the i/C(i) and PsT exhibited that for Nor, PsT appeared after the peak was reached, and for ER1 and ER2, PsT appeared at the beginning of the peak. That is, it was shown that if i/C(i) exceeded the predetermined threshold after PsT, presence of ER could be determined.

Figs. 6C to 8C show that a portion where Z(i) ≤ Zs held and i/C(i) resided within the peak after PsT was the ER portion. This relationship suggests that the following two methods can avoid erroneous detection of the coagulation reaction end point due to ER: a first method is to adopt the coagulation reaction end point if i/C(i) does not exceed the threshold when Z(i) ≤ Zs holds; and a second method is to begin a search after the peak of i/C(i) is reached, and adopt the coagulation reaction end point when Z(i) ≤ Zs holds.

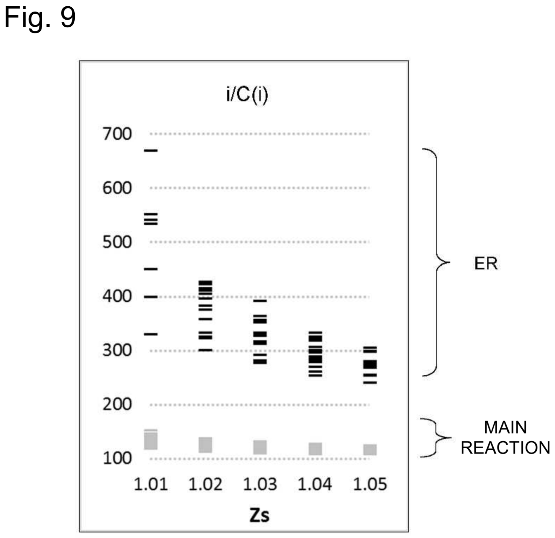

Fig. 9 illustrates a distribution of i/C(i) at the time point T100 at which Z(i) reached the threshold Zs (Zs ranging from 1.01 to 1.05 at 0.01 increments) for each of specimens of ER1 and ER2, that is, T100/C (T100) with respect to different Zs. In the diagram, each black plot represents i/C(i) at a time point when Z(i) reached the threshold Zs for the first time in ER, and each gray plot represents i/C(i) at a time point when Z(i) reached the threshold Zs for the first time after the main reaction began. The distribution regions of the black and gray plots are separated. Accordingly, it was shown that ER can be detected based on i/C(i).

3) Calculation of coagulation time based on Z(i)

The coagulation reaction P(T100) at the coagulation reaction end time point T100 obtained in accordance with the above 1.5) was obtained, and the time point at which P(i) was 50% of P(T100) was assumed as the coagulation time. Note that this corresponds to T50 obtained in the above 1.5).

4) Calculation of coagulation time based on C(i)

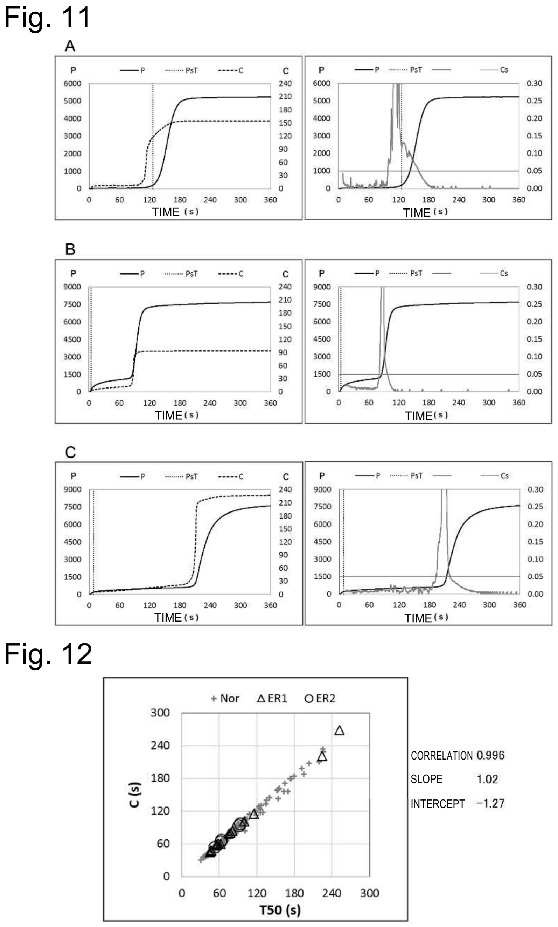

Figs. 10A to 10C illustrate C(i) and i/C(i) for Nor, ER1, and ER2, respectively. In the diagram, C(i) is represented as C. The dotted line along the Y-axis in each diagram indicates the time point PsT at which P(i) exceeds the threshold Ps (= 200). C(i) indicates the time point at which the peak i/C(i) was finished, that is, it rapidly increased at a time point when the main reaction rose, and subsequently leveled off. It is suggested that the leveling off after the steep increase of C(i), for example, the reduction in change rate, was adopted as an indicator, with which coagulation time could be calculated. As an example of the indicator of the change rate of C(i), 10 points before and after i were used and the intra-interval average slope of C(i) was calculated. After the time point PsT when P(i) exceeded the threshold Ps (= 200), a time point when the slope ≤ threshold Cs (= 0.05) holds for the first time was assumed as the coagulation time. Figs. 11A to 11C illustrate C(i) and its slope for Nor, ER1, and ER2, respectively, together with P(i). In the diagram, P(i) and C(i) are represented respectively as P and C, the dotted line along the Y-axis indicates the time point PsT at which P(i) exceeded the threshold Ps (= 200), and the dotted line along the X-axis indicates the threshold Cs. C(i) steeply increased around the time point at which the main reaction of P(i) rose and subsequently leveled off as the main reaction approached the end. The slope of C(i) gradually decreases as the main reaction approached the end.

The coagulation time based on C(i) obtained as described above was plotted against the coagulation time based on Z(i) obtained in the above 3). As illustrated in Fig. 12, irrespective of the specimen group, the coagulation times obtained by both the methods had a high correlation. By any of the methods, it was exhibited that substantially equivalent coagulation times were obtained.

**Claims**

1.   A method for analyzing a blood coagulation reaction, the method comprising:

(1) acquiring P(i) which is a blood coagulation reaction of a blood specimen, where i denotes the number of measurement points or time;
(2) acquiring t1 and t2 from the P(i),

where both t1 and t2 represent the number of measurement points or time, t1 = i, and t2 < t1, and t1 and t2 satisfy an equation: P(t2) = P(t1) × X%, where

the P(t1) and the P(t2) respectively represent blood coagulation reactions of the blood specimen at t1 and t2, and
X ranges from 20 to 70; and

(3) acquiring Rt which represents a ratio of t1 to t2.

2. The method according to claim 1, further comprising evaluating whether the P(i) is a true blood coagulation reaction or not based on the Rt.

3. The method according to claim 1, further comprising evaluating that the P(i) is an early reaction when the Rt is greater than a predetermined value.

4. The method according to claim 1, further comprising repeating steps from the (1) to (3) with i = i + k (where k > 0) until the Rt reaches or becomes smaller than a predetermined value to newly acquire P(i), t1, t2, and Rt.

5. The method according to claim 4, further comprising repeating acquisition of P(i) as i increases until a coagulation reaction end point is detected or a blood coagulation time is calculated, after Rt equal to or smaller than the predetermined value is acquired.

6. The method according to claim 5, further comprising:

   acquiring Z(i) from the P(i), wherein the Z(i) is an accumulated ratio,
   where

$$Z(i) = Pb(i)/Pa(i),$$

   Pa(i) is a sum of P(i - m) to P(i - w),
   Pb(i) is a sum of P(i + w) to P(i + m),
   where i denotes the number of measurement points or time,
   in a case where i indicates the number of measurement points, w = 1, and m is an integer ranging from 10 to 30,
   in a case where i denotes time, w denotes a measurement time interval of the blood coagulation reaction, and m ranges from 1 to 3 seconds; and

   acquiring the P(i) as the coagulation reaction end point, when the Z(i) is a predetermined threshold Zs or smaller.

7. The method according to claim 6, further comprising calculating the blood coagulation time, based on the coagulation reaction end point.

8. The method according to claim 5, further comprising:

   acquiring C(i) from the P(i), wherein the C(i) satisfies an equation: P(C(i)) = P(i) × X%, where X ranges from 20 to 70; and
   calculating the blood coagulation time, based on the acquired C(i).

9. The method according to claim 5, further comprising:

   calculating coagulation reaction rate data from the acquired P(i); and
   calculating the blood coagulation time, based on the coagulation reaction rate data.

10. The method according to claim 1, further comprising repeating the step (1) until the P(i) reaches or exceeds a predetermined threshold Ps.

11. The method according to claim 4, further comprising finishing the repeating of the steps and acquiring a result that the blood coagulation reaction of the blood specimen is not detected, when the i reaches a predetermined measurement end point and the Rt does not reach or is not smaller than the predetermined value.

12. The method according to claim 5, further comprising finishing the repeating of the steps and acquiring a result that the blood coagulation reaction of the blood specimen is not finished, when the i reaches a predetermined measurement end point and the coagulation reaction end point is not detected or the coagulation time is not calculated.

13. The method according to claim 5, further comprising stopping acquiring the P(i), when the coagulation reaction end point is detected or the blood coagulation time is calculated.

**14.** The method according to any one of claims 7 to 9, further comprising outputting the calculated blood coagulation time.

# Fig. 1

| | |
|---|---|
| START MEASUREMENT | S1 |
| DETECT REACTION | S2 |
| WHEN ER IS DETECTED, WAIT FOR MAIN REACTION | S3 |
| CALCULATE COAGULATION TIME FROM MAIN REACTION | S4 |
| (DETERMINE PRESENCE OR ABSENCE OF ERROR IN REACTION CURVE) | S5 |
| OUTPUT MEASUREMENT RESULT | S6 |
| FINISH MEASUREMENT | S7 |

Fig. 2A

```
          START

            │
    ┌───────▼───────┐ S1
    │  ACQUIRE P(i) │
    └───────┬───────┘
            │
          ╱─┴─╲
        ╱   IS   ╲      S2
      ╱ MEASUREMENT ╲ ──── Yes ──────┐
        ╲ FINISHED? ╱                 │
          ╲─┬─╱                       │
           No                         │
          ╱─┴─╲                       │
    No  ╱       ╲ S3                   │
    ◄──╱ P(i) > Ps ╲                   │
        ╲         ╱                    │
          ╲─┬─╱                        │
           Yes                         │
    ┌───────▼───────┐ S4               │
    │ CALCULATE C(i)│                  │
    └───────┬───────┘                  │
          ╱─┴─╲                        │
    No  ╱        ╲ S5                   │
    ◄──╱ i/C(i) ≤ S ╲                  │
        ╲          ╱                    │
          ╲─┬─╱                         │
           Yes                          │
    ┌───────▼───────┐ S6                │
    │ CALCULATE Z(j)│                   │
    └───────┬───────┘                   │
          ╱─┴─╲                         │
    No  ╱        ╲ S7                    │
    ◄──╱ Z(j) ≤ Zs ╲                    │
        ╲         ╱                      │
          ╲─┬─╱                          │
           Yes                           │
    ┌───────▼───────┐ S8                 │
    │   CALCULATE   │                    │
    │COAGULATION TIME│                   │
    └───────┬───────┘                    │
            ◄────────────────────────────┘
    ┌───────▼───────┐ S9
    │OUTPUT MEASUREMENT│
    │    RESULT     │
    └───────┬───────┘
          END
```

START

ACQUIRE P(i) — S1

IS MEASUREMENT FINISHED? — S2 — Yes / No

$P(i) > Ps$ — S3 — No / Yes

CALCULATE C(i) — S4

$i/C(i) \le S$ — S5 — No / Yes

CALCULATE Z(j) — S6

$Z(j) \le Zs$ — S7 — No / Yes

CALCULATE COAGULATION TIME — S8

OUTPUT MEASUREMENT RESULT — S9

END

# Fig. 2B

```
                    ┌─────────────┐
                    │   START     │
                    └─────────────┘
                          │
    ┌─────────────────────┤
    │     ┌───────────────────────────┐
    │     │      ACQUIRE P(i)         │ S1
    │     └───────────────────────────┘
    │                 │
    │              ◇ IS          S2
    │         MEASUREMENT ─────── Yes ──────────┐
    │           FINISHED? ◇                     │
    │                 │ No                      │
    │                 │                         │
    │     No ◇   P(i) > Ps  ◇  S3               │
    ├─────────                                  │
    │                 │ Yes                     │
    │     ┌───────────────────────────┐         │
    │     │      CALCULATE C(i)       │ S4      │
    │     └───────────────────────────┘         │
    │                 │                         │
    │     No ◇   i/C(i) ≤ S  ◇  S5              │
    ├─────────                                  │
    │                 │ Yes                     │
    │     ┌───────────────────────────┐         │
    │     │  CALCULATE SLOPE OF       │ S6      │
    │     │    TANGENT OF C(j)        │         │
    │     └───────────────────────────┘         │
    │                 │                         │
    │          ◇  SLOPE                         │
    │      OF TANGENT ≤ ◇ S7                    │
    │  No    THRESHOLD                          │
    └─────────                                  │
                      │ Yes                     │
          ┌───────────────────────────┐         │
          │      CALCULATE            │ S8      │
          │  COAGULATION TIME         │         │
          └───────────────────────────┘         │
                      │ ◄───────────────────────┘
          ┌───────────────────────────┐
          │  OUTPUT MEASUREMENT       │ S9
          │       RESULT              │
          └───────────────────────────┘
                      │
              ┌─────────────┐
              │    END      │
              └─────────────┘
```

Fig. 3

EP 4 650 782 A1

**1**

**30** MEASUREMENT UNIT

CONTROL CIRCUIT

SPECIMEN CONTAINER

REAGENT CONTAINER

SPECIMEN PROBE

REAGENT PROBE

LIGHT SOURCE → REACTION CONTAINER → DETECTOR

DATA PROCESSING CIRCUIT

**10** CONTROL UNIT

CPU

MEMORY

STORAGE

COMMUNICATION I/F

**20** OPERATION UNIT

UI

**40** OUTPUT UNIT

DISPLAY DEVICE

Fig. 4

Fig. 5

Fig. 6

Fig. 7

Fig. 8

## Fig. 9

Fig. 10

# Fig. 11

# Fig. 12

## INTERNATIONAL SEARCH REPORT

| International application No. |
| --- |
| **PCT/JP2024/000586** |

| | |
| --- | --- |
| **A.** | **CLASSIFICATION OF SUBJECT MATTER** |

*G01N 33/86*(2006.01)i

FI: G01N33/86

According to International Patent Classification (IPC) or to both national classification and IPC

| | |
| --- | --- |
| **B.** | **FIELDS SEARCHED** |

Minimum documentation searched (classification system followed by classification symbols)

G01N33/86

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| A | JP 2003-169700 A (SYSMEX CORPORATION) 17 June 2003 (2003-06-17)<br>claim 1 | 1-14 |
| A | JP 2018-072156 A (SYSMEX CORPORATION) 10 May 2018 (2018-05-10)<br>claim 1 | 1-14 |
| A | WO 2021/132552 A1 (SEKISUI MEDICAL CO., LTD.) 01 July 2021 (2021-07-01) | 1-14 |

☐ Further documents are listed in the continuation of Box C.　　☑ See patent family annex.

| | |
| --- | --- |
| * | Special categories of cited documents: |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **15 March 2024** | **02 April 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/000586**

| Patent document cited in search report | | | Publication date (day/month/year) | Patent family member(s) | | | Publication date (day/month/year) |
|---|---|---|---|---|---|---|---|
| JP | 2003-169700 | A | 17 June 2003 | US | 2003/0138962 | A1 | |
| | | | | claim 1 | | | |
| | | | | EP | 1316802 | A2 | |
| JP | 2018-072156 | A | 10 May 2018 | US | 2018/0120292 | A1 | |
| | | | | claim 1 | | | |
| | | | | EP | 3315972 | A1 | |
| WO | 2021/132552 | A1 | 01 July 2021 | US | 2023/0341423 | A1 | |
| | | | | EP | 4083630 | A1 | |
| | | | | CN | 114829943 | A | |

Form PCT/ISA/210 (patent family annex) (July 2022)

**EP 4 650 782 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003169700 A
- JP 2010217059 A
- JP 2007107889 A
- WO 2021132552 A
- WO 2021206107 A
- JP 2005519267 A

**Non-patent literature cited in the description**

- *Clinical & Laboratory Haematology*, 2002, vol. 24 (6), 321-327